# EUROPÄISCHE PATENTANMELDUNG

(11) **EP 0 786 324 A1**
(43) Veröffentlichungstag der Anmeldung: **30.07.1997**
(21) Anmeldenummer: 96100970.1
(22) Anmeldetag: 24.01.1996
(51) Int. Cl.: B29C 65/56, F16L 47/02, F16L 31/02, A61M 39/12

(54) **Verfahren zum Verbinden eines medizinischen Schlauchs an einem Adapter aus Kunststoff**

(71) Anmelder: B. BRAUN MELSUNGEN AG, 34212 Melsungen (DE)
(72) Erfinder: Fuchs, Juergen, D-34308 Bad Emstal (DE)
(74) Vertreter: Selting, Günther, Dipl.-Ing.

(57) **Zusammenfassung**

Zum klebemittelfreien und lösungsmittelfreien Befestigen eines Schlauch (12) mit einem Adapter (10) aus Kunststoff wird der Schlauch (12) auf ein Stützrohr (13) aufgeschoben, das von einem Mantelrohr (14) umgeben ist. Durch Formwerkzeuge (17,18) erfolgt eine thermische Verformung des Mantelrohres unter Druck, wobei das Schlauchende auf dem Stützrohr (13) mechanisch festgeklemmt wird. Gleichzeitig kann durch die Erwärmung eine Verschweißung der Materialien von Adapter (10) und Schlauch (12) erfolgen. Nach Beendigung der Stromzufuhr wird die Schweißverbindung per Luftstrahl gekühlt. Für diesen Arbeitsablauf wird etwa der gleiche Zeitaufwand benötigt, wie bei einer Verklebung jedoch wird der Klebstoff eingespart und die Verbindung ist sicherer als eine Klebeverbindung.

## Beschreibung

Die Erfindung betrifft ein Verfahren zum Befestigen eines Schlauchs für die Übertragung medizinischer Flüssigkeiten an einem aus Kunststoff bestehenden Adapter.

Üblicherweise werden medizinische Druck- oder Infusionsleitungen an einem aus Kunststoff bestehenden Adapter mittels einer Klebeverbindung befestigt, damit die Schlauchverbindung absolut dicht sowie druck- und reißfest ist. Klebeverbindungen haben jedoch den Nachteil, daß sie eine bestimmte Materialauswahl für Schlauch und Adapter erfordern, da der Klebstoff an den beiden Materialien sicher haften muß. Kunststoffe, die an sich für den Adapter oder Schlauchansatz geeignet sind, wie z.B. Polyethylen oder Polypropylen, lassen sich nicht verkleben. Darüberhinaus ist der Einsatz von Kleber unter Umständen schädlich für das mit der Herstellung der Schlauchverbindung befaßte Personal. Es sind Ekzeme, Allergien sowie Haut- und Augenreizungen nicht auszuschließen.

Aus EP 0 450 330 A1 ist ein Verfahren zur Anbringung eines nicht-klebefähigen Schlauchs an einem klebefähigen Adapter bekannt, bei dem das mit einem abstehenden Flansch versehene Schlauchende in eine Bohrung des Adapters eingeführt und anschließend der Raum zwischen dem Schlauch und der Adapterbohrung mit einem Klebstoff gefüllt wird, der sich mit dem Adapter verbindet und den Schlauch durch Zurückhalten des Flansches festhält. Mit diesem Verfahren lassen sich zwar nicht-klebefähige Schläuche verarbeiten, jedoch kann auf Klebemittel oder ähnliche härtende Substanzen, die die oben geschilderten Nachteile aufweisen, nicht verzichtet wird.

Aus DE 42 02 228 C2 ist ein Verfahren bekannt, bei dem das Ende eines Schlauchs zur Erzeugung eines abstehenden Flansches verformt wird. In das erwärmte Schlauchende wird Lösungsmittel eingebracht und das Schlauchende wird bei noch vorhandenem Lösungsmittel auf das Stützrohr des Adapters aufgeschoben. Bei diesem Verfahren wird der erwärmte Schlauch mit dem Lösungsmittel angelöst, um sich mit dem Stützrohr des Adapters zu verbinden. Der in dem Mantelrohr verbleibende Ringraum wird mit einem Kunstharz ausgefüllt. Auch dieses Verfahren arbeitet mit gesundheitsschädlichen und umweltbelastenden Chemikalien.

Der Erfindung liegt die Aufgabe zugrunde, ein Verfahren zum Befestigen eines Schlauchs sowie eine Schlauchverbindung anzugeben, die eine umweltfreundliche und problemlos durchführbare Verbindungstechnik ermöglichen.

Die Lösung dieser Aufgabe erfolgt mit dem Verfahren des Patentanspruchs 1 und der Schlauchverbindung des Patentanspruchs 5.

Bei dem erfindungsgemäßen Verfahren erfolgt durch thermisches Verformen des Mantelrohres ein Festklemmen des Schlauchendes zwischen Mantelrohr und Stützrohr in der Weise, daß die Verbindung sich zerstörungsfrei nicht wieder lösen läßt. Durch diese Verbindungsart ist es außerdem möglich, Kunststoffe für den Schlauch sowie den Adapter auszuwählen, die sich nicht verkleben lassen, z.B. Polyethylen, Polypropylen oder Polyolefine. Insbesondere können Schlauch und Adapter aus demselben nicht-klebefähigen Material bestehen, wobei beide Teile ohne Fremdstoffe miteinander verbunden werden. Dies hat den Vorteil, daß das betreffende medizinische Gerät, bei dem es sich in der Regel um Einmalartikel handelt, die nach Gebrauch entsorgt werden müssen, sortenrein sortiert werden kann. Für die thermische Verformung wird etwa der gleiche Zeitaufwand benötigt wie bei einer Verklebung, jedoch wird der Klebstoff eingespart. Außerdem ist die Verbindungstechnik sicherer als die Klebetechnik.

Vorzugsweise erfolgt bei der thermischen Verformung des Mantelrohrs zugleich ein Verschmelzen der Materialien von Adapter und Schlauch. Auf diese Weise werden die Materialien vollflächig miteinander verschweißt, was zu einer besonders guten Abdichtung führt.

Das thermische Verformen erfolgt zweckmäßigerweise durch Widerstandsheizung mit elektrisch leitenden Formwerkzeugen. Eine solche Widerstandsheizung kann bei der Massenproduktion wirksam eingesetzt werden.

Das erfindungsgemäße Verfahren und die erfindungsgemäße Schlauchverbindung sind speziell für medizinische Anwendungen unter sterilen Bedingungen vorgesehen. Sie eignen sich für Infusionsleitungen, Tropfkammern, Ernährungssonden, Perfusorleitungen, Katheter u.dgl. Alle diese Anwendungen erfordern Schläuche und Schlauchverbindungen von hoher Dichtigkeit und Funktionssicherheit. Andererseits sind die Schläuche Einmalartikel, die nach Gebrauch entsorgt werden und daher eine einfache und kostengünstige Herstellungstechnik erfordern.

Im folgenden werden Ausführungsbeispiele der Erfindung unter Bezugnahme auf die Zeichnungen näher erläutert.

Es zeigen:
- Fig. 1: die Anwendung des erfindungsgemäßen Verfahrens beim Anschluß eines Schlauchs an eine Tropfkammer,
- Fig. 2: einen Schlauchansatz, der nach dem Verfahren mit einem Schlauch verbunden ist,
- Fig. 3: eine andere Ausführungsform des Unterteils einer Tropfkammer nach dem Verbinden mit einem Schlauch,
- Fig. 4: eine Seitenansicht eines Dreiwegehahns für den Anschluß an medizinische Schlauchleitungen,
- Fig. 5: eine Draufsicht des Dreiwegehahns von Fig. 4, und
- Fig. 6: einen Längsschnitt durch das Gehäuse des Dreiwegehahns gemäß der Linie VI-VI von Fig. 5.

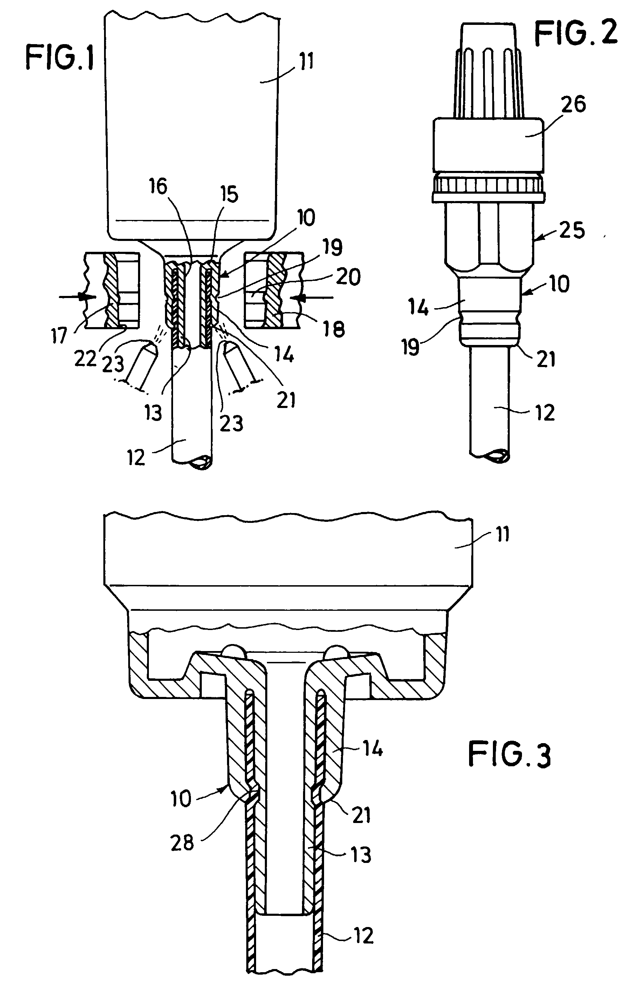

In Fig. 1 ist der Adapter 10 eines medizinischen Gerätes 11 dargestellt, an den ein Schlauch 12 angeschlossen werden soll. Der Adapter 10 weist ein inneres Stützrohr 13 auf, das von einem äußeren Mantelrohr 14 umgeben ist. Das Stützrohr 13 und das Mantelrohr, 14 sind an dem geräteseitigen Ende des Adapters 10 durch eine Stirnwand 15 miteinander verbunden. Durch das Stützrohr 13 hindurch verläuft ein Kanal 16, der mit dem Lumen des Schlauchs 12 in Verbindung steht.

Das medizinische Gerät 11 ist bei dem in Fig. 1 dargestellten Beispiel das Unterteil einer Tropfkammer. Diesem Gerät 11 ist der Adapter 10 einstückig angeformt.

Das Ende des Schlauches 12 ist auf das Stützrohr 13, welches aus dem Mantelrohr 14 heraus vorsteht, aufgeschoben und füllt den Ringraum zwischen Stützrohr 13 und Mantelrohr 14 aus. Mit zwei Formwerkzeugen 17 und 18 wird das Mantelrohr 14 zusammengepreßt. Die Formwerkzeuge 17 und 18 bestehen aus elektrisch leitendem Material und werden durch Widerstandserwärmung aufgeheizt. Die Formwerkzeuge 17 und 18 haben eine Innenkontur, die der beabsichtigten Außenkontur des Mantelrohres 14 entspricht. In das Mantelrohr 14 soll in diesem Fall eine umlaufende Sicke 19 eingeformt werden, an der der Schlauch 12 verstärkt gepreßt wird. Demzufolge ist an den Formwerkzeugen jeweils ein Wulst 20 vorgesehen. Ferner soll am Ende des Mantelrohres 14 eine nach innen gerichtete Bördelung 21 erzeugt werden. Demzufolge haben die Formwerkzeuge 17 und 18 an ihrem äußeren Ende eine Einschnürung 22.

Wenn die beiden Formwerkzeuge 17 und 18 gegen das Mantelrohr gepreßt sind, werden sie durch einen kurzzeitigen Stromstoß erhitzt. Dabei wird der Schlauch 12 mit dem Adapter 10 verschmolzen. Sofort nach Beendigung der Stromzufuhr wird die Schweißverbindung durch Luftstrahlen, die aus Düsen 23 austreten, gekühlt, nachdem die Formwerkzeuge auseinandergefahren sind.

In Fig. 2 ist ein Konnektor 25 für den Anschluß an ein medizinisches Gerät dargestellt. Der Adapter 10 des Konnektors wurde nach dem erfindungsgemäßen Verfahren mit einem Schlauch 12 verbunden. Der Konnektor 25 weist eine Luer-Lock-Kupplung auf, die hier mit einem Verschlußteil 26 verschlossen ist. Man erkennt das Mantelrohr 14 mit der eingeformten Sicke 19 und der an ihrem Ende angeformten Bördelung 21.

Bei dem Ausführungsbeispiel von Fig. 3 ist ebenfalls ein medizinisches Gerät 11 in Form einer Tropfkammer, mit einem einstückig angeformten Adapter 10 versehen, der mit einem Schlauch 12 verbunden ist. Der Schlauch 12 ist auf das Stützrohr 13 aufgeschoben und liegt vollflächig an diesem an. Am Stützrohr 13 ist außen eine umlaufende Vertiefung 28 vorgesehen, in die der Schlauch 12 von dem Bördelrand 21 des Mantelrohres 14 gedrückt wird. Dadurch erfährt der Schlauch 12 eine örtliche Einschnürung, die eine verstärkte Sicherung gegen Herausziehen und Undichtigkeiten darstellt.

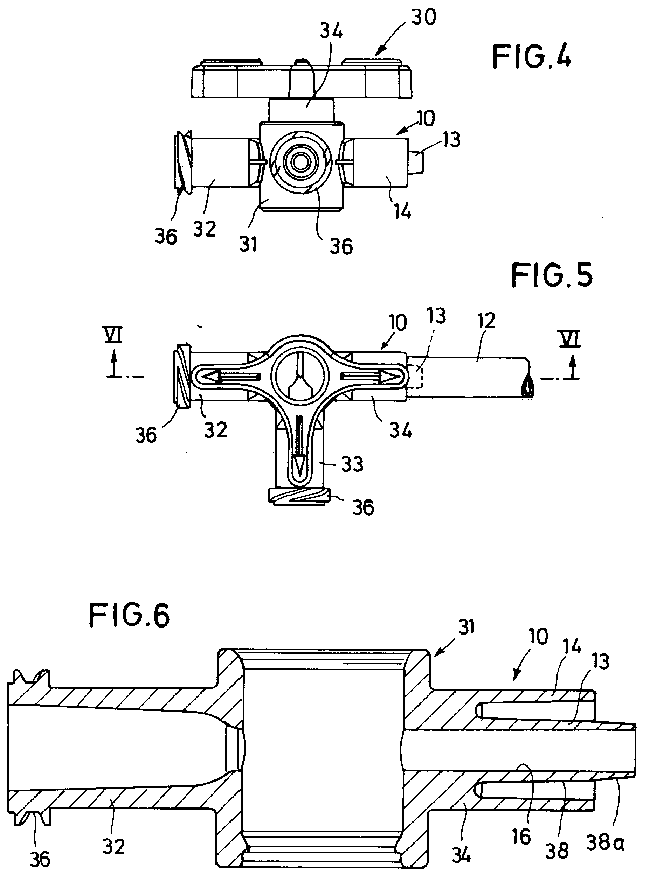

Die Fign. 4 bis 6 zeigen einen Dreiwegehahn 30 mit einem Gehäuse 31, das drei Anschlußstutzen 32,33,34 aufweist, und einem in das Gehäuse 31 hineinragenden Küken 34, das die Anschlußstutzen in wählbarer Kombination untereinander verbindet. Die Anschlußstutzen 32,33 sind mit Luer-Konnektoren 36 ausgestattet, während der Anschlußstutzen 34 als Adapter 10 ausgebildet ist, der mit dem Schlauch 12 fest verbunden ist.

Gemäß Fig. 6 hat das Mantelrohr 14 eine zylindrische Innenwand und eine zylindrische Außenwand während das koaxial im Mantelrohr angeordnete Stützrohr 13 einen zylindrischen Kanal 16 enthält. Die Außenwand 38 des Stützrohres 13 ist konisch ausgebildet und sie weist einen Endabschnitt 38a von verstärkter Konnizität auf, um das Aufschieben des Schlauchendes zu erleichtern.

## Patentansprüche

1. Verfahren zum Befestigen eines Schlauchs (12) für die Übertragung medizinischer Flüssigkeiten an einem aus Kunststoff bestehenden Adapter (10), bei welchem das Schlauchende auf ein von einem Mantelrohr (14) umgebenes Stützrohr (13) des Adapters (10) aufgeschoben und zwischen Stützrohr (13) und Mantelrohr (14) fixiert wird,
**dadurch gekennzeichnet,**
daß das Fixieren des Schlauchendes durch thermisches Verformen des Mantelrohres (14) erfolgt, wobei das Schlauchende mechanisch zwischen Mantelrohr (14) und Stützrohr (13) eingeklemmt wird.

2. Verfahren nach Anspruch 1, dadurch gekennzeichnet, daß bei dem thermischen Verformen des Mantelrohres (14) die Materialien von Adapter (10) und Schlauch (12) miteinander verschmolzen werden.

3. Verfahren nach Anspruch 1 oder 2, dadurch gekennzeichnet, daß das thermische Verformen durch Widerstandserhitzung mit elektrisch leitenden Formwerkzeugen (17,18) erfolgt.

4. Verfahren nach einem der Ansprüche 1 bis 3, dadurch gekennzeichnet, daß der Schlauch (12) beim Verformen des Mantelrohres (14) in mindestens eine Vertiefung (28) des Stützrohres (13) eingepreßt wird.

5. Schlauchverbindung an einem medizinischen Gerät (11) oder für den Anschluß an ein medizinisches Gerät, mit einem Adapter (10), der ein Stützrohr (13) und ein dieses umgebendes Mantelrohr (14) aufweist, wobei das Schlauchende zwischen Stützrohr (13) und Mantelrohr (14) eingeschlossen ist, dadurch gekennzeichnet, daß das Schlauchende zwischen Stützrohr (13) und Mantelrohr (14) durch mechanisches Einklemmen, und ggf. zusätzlich durch Materialverschweißung, druckdicht und reißfest gehalten ist.

6. Schlauchverbindung nach Anspruch 5, dadurch gekennzeichnet, daß das Stützrohr (13) mindestens eine umlaufende Vertiefung (28) aufweist, und daß das Mantelrohr (14) eine Sicke oder an seinem Ende eine Bördelung (21) aufweist, die den Schlauch (12) in die Vertiefung (28) drückt.

7. Schlauchverbindung nach Anspruch 5 oder 6, dadurch gekennzeichnet, daß das Mantelrohr (14) eine nach innen gerichtete Sicke (19) aufweist, die den Schlauch verstärkt zusammenpreßt.

8. Schlauchverbindung nach einem der Ansprüche 5 bis 7, dadurch gekennzeichnet, daß der Adapter (10) und der Schlauch (12) aus gleichem Material bestehen.
